# EUROPEAN PATENT SPECIFICATION

(11) **EP 3 707 513 B1**
(45) Date of publication and mention of the grant of the patent: **07.02.2024**
(21) Application number: 18803353.4
(22) Date of filing: 06.11.2018
(51) Int. Cl.: G01N 33/68

(54) **METHOD FOR ESTABLISHING THE PRESENCE AND PROGRESSION OF NEURODEGENERATIVE DISEASE**
VERFAHREN ZUR FESTSTELLUNG DES VORLIEGENS UND FORTSCHREITENS EINER NEURODEGENERATIVEN ERKRANKUNG
PROCÉDÉ D'ÉTABLISSEMENT DE LA PRÉSENCE ET DE LA PROGRESSION D'UNE MALADIE NEURODÉGÉNÉRATIVE

(30) Priority: 07.11.2017 IT 201700126773
(43) Date of publication of application: 16.09.2020
(73) Proprietor: Fondazione Istituto Italiano di Tecnologia, 16163 Genova (IT); Fondazione Santa Lucia, 00179 Roma (IT)
(72) Inventor: ARMIROTTI, Andrea, 16138 Genova (IT); REGGIANI, Angelo, 16036 Recco (GE) (IT); SPALLETTA, Gianfranco, 00147 Roma (IT)
(74) Representative: Bianchetti & Minoja with Trevisan & Cuonzo IPS SRL
(86) International application number: PCT/EP2018/080259
(87) International publication number: WO 2019/091952

(56) References cited:
- EP-A1- 2 950 102
- BASIT ABDUL ET AL: "Ion mobility mass spectrometry enhances low-abundance species detection in untargeted lipidomics", METABOLOMICS, SPRINGER NEW YORK LLC, US, vol. 12, no. 3, 8 February 2016 (2016-02-08), pages 1-10, XP035936366, ISSN: 1573-3882, DOI: 10.1007/S11306-016-0971-3 [retrieved on 2016-02-08]
- XIANLIN HAN ET AL: "Metabolomics in Early Alzheimer's Disease: Identification of Altered Plasma Sphingolipidome Using Shotgun Lipidomics", PLOS ONE, vol. 6, no. 7, 1 July 2011 (2011-07-01), page e21643, XP055351379, DOI: 10.1371/journal.pone.0021643
- ARMIROTTI ANDREA ET AL: "Sample preparation and orthogonal chromatography for broad polarity range plasma metabolomics: Application to human subjects with neurodegenerative dementia", ANALYTICAL BIOCHEMISTRY, vol. 455, 4 April 2014 (2014-04-04), pages 48-54, XP028659747, ISSN: 0003-2697, DOI: 10.1016/J.AB.2014.03.019
- GUAN ZHIZHONG ET AL: "Decrease and structural modifications of phosphatidylethanolamine plasmalogen in the brain with Alzheimer disease", JOURNAL OF NEUROPATHOLOGY AND EXPERIMENTAL NEUROL, LIPPINCOTT WILLIAMS AND WILKINS, NEW YORK, NY, vol. 58, no. 7, 1 July 1999 (1999-07-01), pages 740-747, XP008128120, ISSN: 0022-3069
- ALFRED N. FONTEH ET AL: "Alterations in cerebrospinal fluid glycerophospholipids and phospholipase A 2 activity in Alzheimer's disease", JOURNAL OF LIPID RESEARCH, vol. 54, no. 10, 1 October 2013 (2013-10-01), pages 2884-2897, XP055470036, US ISSN: 0022-2275, DOI: 10.1194/jlr.M037622
- YOKO TAJIMA ET AL: "Lipidomic analysis of brain tissues and plasma in a mouse model expressing mutated human amyloid precursor protein/tau for Alzheimer's dis", LIPIDS IN HEALTH AND DISEASE, BIOMED CENTRAL, LONDON, GB, vol. 12, no. 1, 9 May 2013 (2013-05-09), page 68, XP021151111, ISSN: 1476-511X, DOI: 10.1186/1476-511X-12-68

## Description

The present invention relates to an *in vitro* method for establishing the presence, progression or risk of onset of neurodegenerative disease, and biomarkers useful for said purpose.

The method according to the invention is based on determination of the concentration of lipid markers in an individual's serum or plasma sample, and it is able to discriminate, with a high degree of specificity, individuals suffering from neurodegenerative disease, and to predict its progression in patients with a positive diagnosis of neurodegenerative disease who have already received, or are currently receiving, pharmacological treatment.

### Introduction

Parkinson's disease and Alzheimer's disease (PD and AD respectively) are neurodegenerative disorders that affect motor functions and cognitive/neuropsychiatric performances such as memory, speech, executive functions, mood and thought. With the progressive ageing of the population, the incidence of these conditions is constantly increasing, giving rise to a major burden on healthcare systems.

For example, epidemiological data suggest that in subjects above 60 years of age, as many as 1% of the worldwide population in the Western countries are PD sufferers, with a rising incidence associated with age. Moreover, PD causes over 100,000 deaths a year worldwide. On the basis of current data, it has been estimated that every year in the European countries, 9 out of 1000 people between 75 and 79 years of age may develop AD. At the present time, *in vivo* diagnosis and prognosis of PD and AD are essentially based on clinical assessment, cerebrospinal fluid imaging and/or radiological and nuclear medicine (mainly magnetic resonance and positron-emission tomography). However, none of these approaches is sensitive enough to provide a definite early diagnosis, because the symptoms of brain damage must be well-established before making a final diagnosis *in vivo.* At present, both PD and AD can only be diagnosed when clinical symptoms are already detectable, ie. when the neuronal damage is at an advanced stage and the therapeutic options available to doctors (neurologists, psychiatrists and geriatricians) are greatly reduced. At present, no molecular biomarkers for PD and AD are available in clinical practice, although several have been proposed. It would therefore be highly desirable to have a circulating molecule, measurable with a simple blood test and correlated with the progression and severity of neurodegeneration, which could serve as an early marker of the onset of the disease.

### State of the art

Yoko Tajima et al.: "Lipidomic analysis of brain tissues and plasma in a mouse model expressing mutated human amyloid precursor protein/tau for Alzheimer's disease", LIPIDS IN HEALTH AND DISEASE, BIOMED CENTRAL, LONDON, GB, vol. 12, no. 1, 9 May 2013 (2013-05-09), page 68, discloses decreases in plasma lipid levels in an Alzheimer mouse model (tab 3).

WO2007/050318 describes a method of correlating the lipid profile with the progression of a central nervous system (CNS) disorder, such as a psychiatric or neurodegenerative disorder. The metabolites used as biomarkers for CNS disorders include phosphatidylethanolamines and phosphatidylcholine.

EP1482920 relates to the use of phospholipid mixtures containing N-acetylphosphatidylethanolamines (NAPEs) and/or phospholipid mixtures containing N-acetylethanolamines (NAEs) together with phosphatidic acids (PAs) and/or lysophosphatidic acids (LPAs) for the preparation of medicaments having anorexic activity or of medicaments or foodstuffs for the treatment of various disorders, such as AD, PD and senile dementia.

The article Coulon, D. et al. - Occurrence, biosynthesis and functions of N-acylphosphatidylethanolamines (NAPE): Not just precursors of N-acylethanolamines (NAE) describes the role of bioactive N-acylphosphatidylethanolamine molecules in various physiological processes. The article shows that NAPE levels increase strongly in response to stress conditions involving degenerative membrane changes. In particular, NAPE levels strongly increased in the cortex, hippocampus and inferior colliculus.

EP2950102 describes a method for establishing the probability with which a patient with a cognitive disorder may develop AD. The method is based on detection of the levels of various biomarkers, including ceramide, in blood, serum or plasma samples.

### Description of figures

**Figure 1** Comparison of plasma levels of NAPE in AD patients and healthy controls (***= p<0.001 2-tailed Student's "t" test).
**Figure 2** ROC curves for NAPEs P16:0-22:6/N18:0 and 18:0-22:6/N16:0, showing the best ability to discriminate between male and female PD patients compared with the corresponding healthy controls.
**Figure 3** Left: results of a two-tailed Mann-Whitney t-test comparing NAPE 18:0-20:4/N18:0 levels in the plasma of 62 healthy female subjects and 14 PD patients under 50 years of age (P=0.0001). Right: ROC curve for NAPE 18:0-20:4/N14:0 plasma levels in female PD patients under 50 years of age by comparison with age-matched healthy controls.
**Figure 4** Results of a two-tailed Mann-Whitney t-test comparing the levels of 7 NAPEs in the plasma of 29 healthy subjects and 30 AD patients.
**Figure 5** Left: results of a two-tailed Mann-Whitney t-test comparing the sum of the NAPE P18:0-22:6-N18:0, 18:0-20:4-N18:0, 16:0-22:6-N18:0 and P16:0-22:6-N18:0 levels in the plasma of healthy volunteers and AD patients (N=30). Right: ROC curve for the same sum of plasma levels in AD patients by comparison with age-matched healthy controls.

### Description of the invention

It has now been discovered that the circulating N-acylphosphatidylethanolamine (NAPE) levels are significantly correlated with the presence of neurodegenerative disease, and are able to predict its onset and progression.

In particular it has been observed that the reduction in the concentration of particular NAPEs in an individual's plasma or serum samples constitutes a useful diagnostic and prognostic indicator or marker, also indicating the risk of onset of neurodegenerative disease.

The invention therefore provides an *in vitro* method for the diagnosis or prognosis of neurodegenerative disease, or for determining the risk that it will develop in a individual, comprising:
a) determination of the concentration of a lipid marker selected from the following N-acylphosphatidylethanolamines (NAPEs): P18:0-22:6/N16:0; P16:0-22:6/N18:0; 18:0-22:6/N16:0; 16:0-22:6/N18:0; 18:0-20:4/N18:0, P18:0-22:6/N18:0, and 18:0-22:6/N18:0, or a combination thereof, in a plasma or serum sample of said individual, and
b) comparison of said concentration with a reference value corresponding to the lipid marker concentration in the plasma or serum of subjects not suffering from same neurodegenerative disease,
wherein a reduction in said concentration detected in the plasma or serum sample of the individual tested compared with the reference value indicates the presence of neurodegenerative disease, an increased risk of developing it, or its progression.

A further aspect of the invention relates to an *in vitro* method for evaluating the efficacy of a therapeutic treatment of a neurodegenerative disease, comprising determination of the concentration of a lipid marker selected from the following N-acylphosphatidylethanolamines (NAPEs): P18:0-22:6/N16:0; P16:0-22:6/N18:0; 18:0-22:6/N16:0; 16:0-22:6/N18:0; 18:0-20:4/N18:0, P18:0-22:6/N18:0, and 18:0-22:6/N18:0, or a combination thereof, in a plasma or serum sample of an individual, wherein a reduction in said lipid marker concentration during the treatment indicates progression of the disease and therefore lower efficacy of the treatment, whereas an increase in said lipid marker concentration during the treatment indicates a slowing or regression of the disease, and therefore greater efficacy of the treatment.

The NAPEs used as biomarkers according to the invention are represented by the following structural formulas:

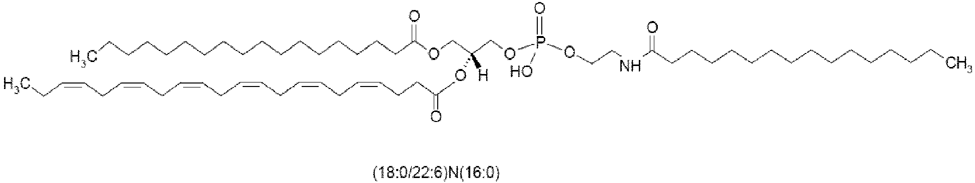

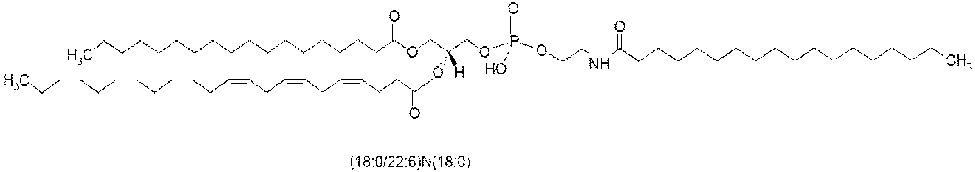

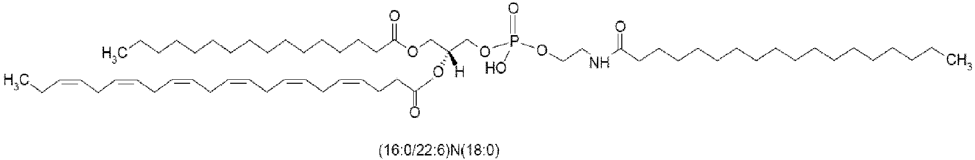

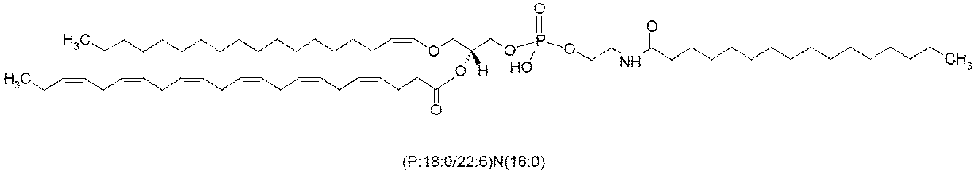

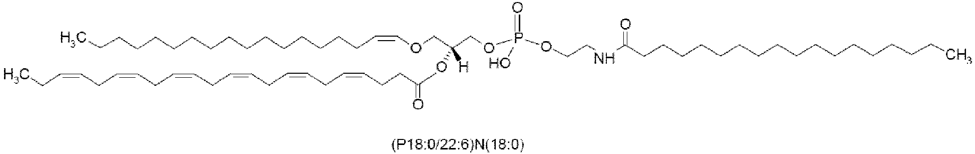

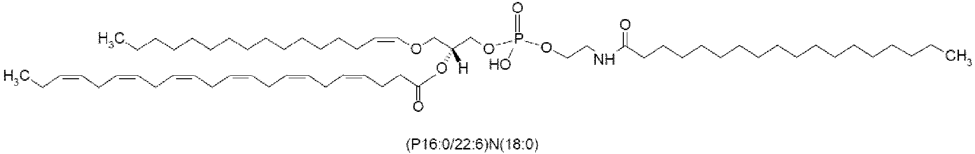

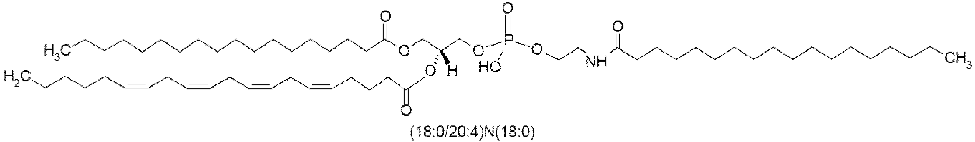

The various NAPEs can be used individually or combined with one another. The skilled person is able to select the NAPE or optimum combination of NAPEs to be used with the method according to the invention on the basis of the type of neurodegenerative disease or its progression.

The reference value compared with which the presence of neurodegenerative disease, its progression or the probability of its onset are determined in the individual tested corresponds to the concentration of NAPE or combination of NAPEs found in the serum or plasma samples of subjects not suffering from the same neurodegenerative disease. To make the diagnostic capacity of the test more effective, the subjects can be clustered by age group or sex.

The skilled person, starting with a group of subjects not suffering from neurodegenerative disease, can determine the most suitable reference value on the basis of predefined specificity and sensitivity parameters, in order to identify the presence of neurodegenerative disease, its progression or the probability of its onset in the individual tested.

Alternatively, the reference value can correspond to the concentration of NAPE, or a combination of NAPEs, found in the same individual at different times. For example, if the individual undergoes pharmacological or other treatment for the neurodegenerative disease, the determination of the NAPE levels and their comparison with the values previously determined during the treatment allows the progression or regression of the disease, and therefore the efficacy of the treatment, to be evaluated.

In a preferred embodiment of the invention, the neurodegenerative disease is PD. In this case, the most effective NAPEs are selected from P18:0-22:6/N16:0; P16:0-22:6/N18:0; 18:0-22:6/N16:0; 18:0-20:4/N18:0 and 18:0-22:6/N18:0.

In particular, NAPE 18:0-20:4/N18:0 has proved to be a more effective PD marker, able to discriminate female individuals with a high level of specificity.

The reference value corresponding to the NAPE concentration in the plasma or serum of subjects not suffering from PD preferably ranges from 1.30 to 3.25 pmol/ml. A NAPE concentration found in the tested individual which is less than said reference values is therefore indicative of the presence, progression or probability of onset of PD, with test specificity margins ranging from 95 to 100% between the two extremes of the range.

In another preferred embodiment of the invention, the neurodegenerative disease is AD. In this case the most effective NAPEs to be used in the method according to the invention are selected from P18:0-22:6/N16:0; P16:0-22:6/N18:0; 16:0-22:6/N18:0; 18:0-20:4/N18:0 and P18:0-22:6/N18:0.

The reference value corresponding to the NAPE concentration in the plasma or serum of subjects not suffering from AD can range from 4.0 to 6.0 pmol/ml. A NAPE concentration found in the tested individual which is less than said reference values is therefore indicative of the presence, progression or probability of onset of Alzheimer's disease, with test specificity margins ranging from 95 to 100% between the two extremes of the range.

The amount of lipid marker in the sample tested can be determined by mass spectrometry techniques coupled with liquid chromatography. Not claimed is a kit for the determination of the concentration of a lipid marker as defined herein from a plasma or serum sample, wherein said kit contains known amounts of one or more of the following NAPEs: P18:0-22:6/N16:0; P16:0-22:6/N18:0; 18:0-22:6/N16:0; 16:0-22:6/N18:0; 18:0-20:4/N18:0, P18:0-22:6/N18:0, and 18:0-22:6/N18:0, which can be used to prepare a calibration curve. The kit can also contain reagents, internal standards, buffers or solvents needed to conduct a NAPE analysis. The kit will also include the detailed analytical parameters required to acquire, analyse and interpret the data.

The invention will be further illustrated in the following experimental part.

### Experimental part

### NAPE synthesis

The three NAPEs (18:0/22:6)N17:0, (18:0/22:6)N16:0 and (18:0/22:6)N18:0 were synthesised and used as internal standards to prepare a calibration curve.

The NAPEs were synthesised according to the protocol described by Guo and colleagues (Guo et al. 2010). Briefly, 0.025 g (0.03 mmol) of 1-stearoyl-2-docosahexanoyl-sn-glycero-3-phosphoethanolamine was dissolved in 2.5 ml chloroform containing 0.0125 g triethylamine in a precooled 7 ml glass vial. An appropriate acyl chloride (16:0, 17:0 or 18:0, 0.12 mmol each) in 0.9 ml chloroform was added dropwise. After the addition was complete, the reaction mixtures were heated to 40°C for 2 h and stirred overnight at room temperature. The reactions were quenched by adding a saturated sodium bicarbonate solution (2.5 ml), and the organic layers were collected and washed with 3.0 ml of 0.01 M hydrochloric acid and 3.0 ml of brine. The reaction mixtures were dehydrated with sodium sulphate, and the product was dried in an N2 evaporator and purified using silica gel chromatography. The purified NAPEs were weighed, and the final amount of NAPE obtained (in mg) was compared with the theoretical amount. The reaction yield ranged from 75 to 80%.

### NAPE analysis

The plasma samples were extracted with ethanol using the above-described exogenous 18:0-22:6-N17:0 NAPE added to a final 0.5 µM concentration to the ethanol extraction mixture. In short: 0.1 ml of plasma was mixed with 1 ml of ethanol. The solution was vortexed for 1 minute, then centrifuged at 3500 rpm at 4°C for 10 minutes. The supernatant was then collected and dried under nitrogen stream. The dried samples were then resuspended in 0.1 ml of 9:1 MeOH/CHCl₃ solution for LC-MS/MS analysis. Targeted analysis of the samples was then carried out on an Acquity UPLC system coupled with a Xevo TQ-MS triple quadrupole mass spectrometer. The NAPEs were separated on an HSS T3 C18 column (2.1 × 50 mm, 1.7 µm) at a flow rate of 0.4 ml/min. The mobile phase consisted of 10 mM ammonium formate (pH 5) in water/acetonitrile (60:40 v/v) as solvent A, and 10 mM ammonium formate (pH 5) in acetonitrile/isopropyl alcohol (10:90 v/v) as solvent B. A linear gradient program was developed for the separation of all metabolites: 0.0-0.5 min 50% B, 0.5-3.5 min 50 to 100% B, and 3.5-4.5 min maintained at 100% B. The column was then reconditioned to 50% B for 1.0 min. The total run time for the analysis was 6 min, and the injection volume was set to 5 µl. The mass spectrometer was operated in the positive ESI mode, and the analytes were quantified using the multiple reaction monitoring parameters reported below:

| Sr. No. | sn-1, sn-2 NAPEs | MRM transitions |
|---|---|---|
| 1 | P18:0-22:6-N16:0 | 1014.8→282.3/635.5 |
| 2 | P16:0-22:6-N18:0 | 1014.8→310.3/607.5 |
| 3 | 18:0-22:6-N16:0 | 1030.8→282.3/651.5 |
| 4 | 16:0-22:6-N18:0 | 1030.88→310.3/623.5 |
| 5 | 18:0-20:4-N18:0 | 1034.8→310.3/627.5 |
| 7 | 18:0-22:6-N18:0 | 1058.8→310.3/651.5 |
| IS | 18:0-22:6-N17:0 | 1044.6→296.4/651.5 |

The capillary voltage was set at 3 kV. The cone voltage and collision energy values were set to 25 V and 20 eV respectively for all transitions. The source temperature was set to 120°C. The desolvation gas and cone gas (N2) flows were set to 800 and 20 L/h respectively. The desolvation temperature was set to 450°C. This method was validated as already described (Basit et al. 2015): 18:0/22:6/N17:0 NAPE, an exogenous molecule not naturally present in plasma, was spiked in naïve human plasma and extracted (N = 3). The quantification limit, recovery, matrix effect and extraction efficiency were evaluated using a calibration curve prepared with the same standard in the 0.05-50 nM concentration range.

### Determination of NAPE concentration in PD and AD patients

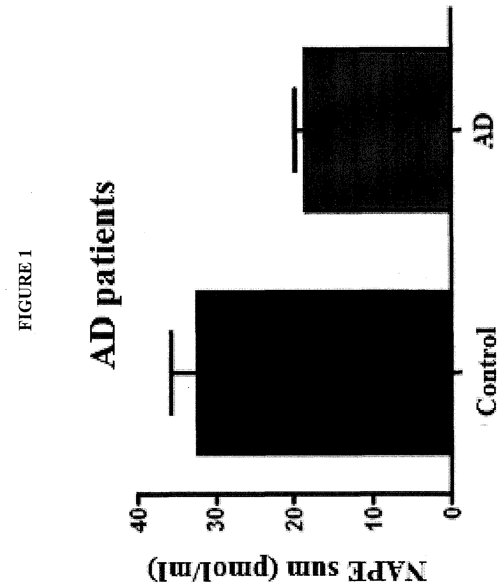

NAPE levels were measured in a set of plasma samples from male and female subjects with a diagnosis of PD by comparison with the plasma of age-matched healthy volunteers. The plasma NAPE levels of a total of 268 PD patients (aged 24 to 88 years) were compared with those of 290 healthy volunteer controls (aged 23 to 92 years). The comparison was made with the two-tailed Mann-Whitney t-test, and the results are set out in Table 1 below and the annexed Figure 1:

**Table 1. Results of comparison of plasma NAPE levels in healthy volunteers and PD patients. The table shows the average % change measured, and the P-value calculated with the two-tailed Mann-Whitney t-test.**

| | 268 PD PATIENTS | | |
|---|---|---|---|
| NAPE | Change | P-value* | Significance |
| P18:0-22:6-N16:0 | -14% | < 0.0001 | *** |
| P16:0-22:6-N18:0 | -28% | < 0.0001 | *** |
| 18:0-22:6-N16:0 | -25% | < 0.0001 | *** |
| 16:0-22:6-N18:0 | -11% | 0.013 | * |
| 18:0-20:4-N18:0 | -21% | < 0.0001 | *** |
| 18:0-22:6-N18:0 | -26% | < 0.0001 | *** |

As indicated, all the NAPEs examined exhibit a statistically significant reduction in the PD group. To establish whether the individual's sex can influence NAPE levels, the data relating to some NAPEs were re-analysed by clustering patients according to sex. As reported in Table 2, while the general trend shows a decrease in NAPE lipids regardless of sex, female subjects show a greater decrease than males.

**Table 2. Results of comparison of plasma levels of NAPE in healthy subjects and PD patients clustered by sex. The table shows the measured average % change, the P-value calculated with the two-tailed Mann-Whitney t-test (*), and the area under curve (AUC) deriving from a ROC analysis with 95% confidence (**). The most significant changes are highlighted.**

| | FEMALE PD SUBJECTS (117)* | | | | MALEPD SUBJECTS (151)* | | | |
|---|---|---|---|---|---|---|---|---|
| NAPE | Change | P-value* | Significance | AUC** | Change | P-value* | Significance | AUC** |
| P18:0-22:6-N16:0 | -19% | < 0.0001 | *** | 0.653 | -10% | 0.104 | ns | 0.557 |
| P16:0-22:6-N18:0 | -37% | < 0.0001 | *** | 0.749 | -14% | 0.010 | * | 0.590 |
| 18:0-22:6-N16:0 | -28% | < 0.0001 | *** | 0.669 | -27% | < 0.0001 | *** | 0.638 |
| 18:0-20:4-N18:0 | -26% | < 0.0001 | *** | 0.696 | -19% | 0.001 | *** | 0.621 |
| 18:0-22:6-N18:0 | -32% | < 0.0001 | *** | 0.698 | -20% | 0.002 | ** | 0.606 |

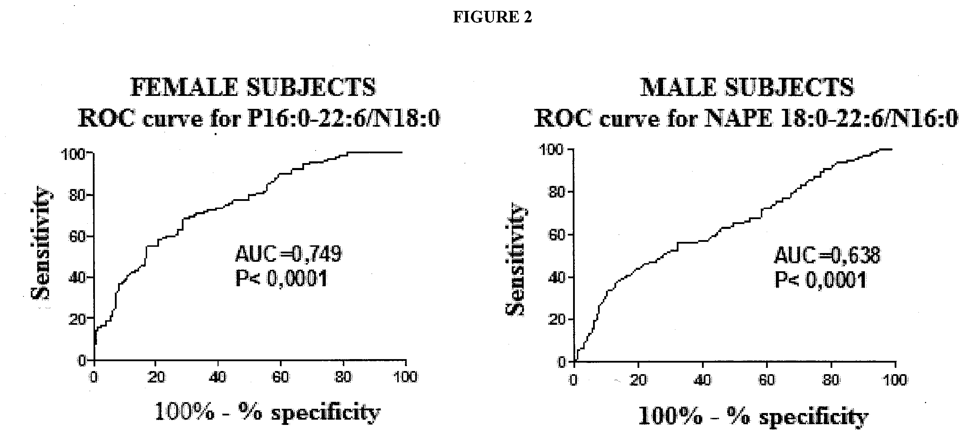

A Receiver Operator Characteristics (ROC) analysis was also performed by plotting the NAPE levels of PD patients (sensitivity) versus those of the healthy controls (1-Specificity). This data analysis approach has become more and more popular in metabolomics, and it has recently been adopted to evaluate the predictive power of a set of plasma phospholipids in the onset of AD (Mapstone, M., et al., Plasma phospholipids identify antecedent memory impairment in older adults. Nat Med, 2014. 20(4): p. 415-8). The observed results are reported in Table 2. Figure 2 reports the ROC curve for the NAPEs showing the best predicting ability for male and female PD patients.

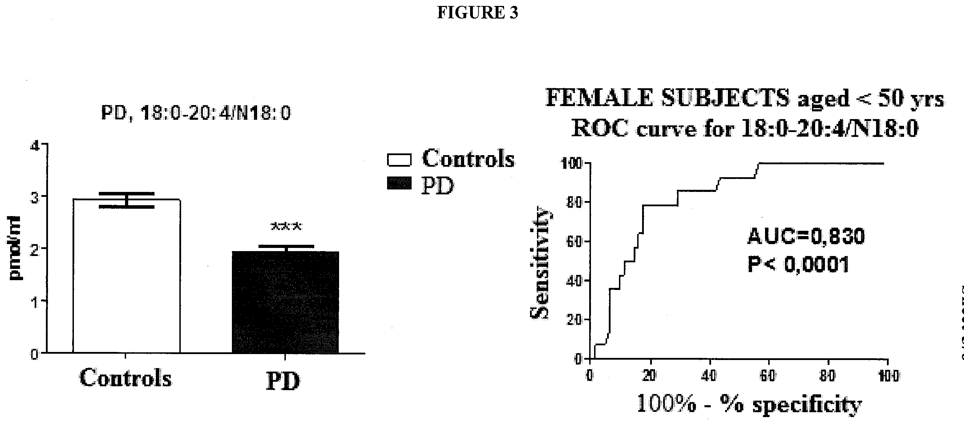

The ROC analysis indicates that NAPEs have, in general, a stronger predicting potential for female than male PD patients. At 95% confidence, female PD patients have lower plasma levels of P16:0-22:6/N18:0 than about 75% of the corresponding healthy controls, while male PD patients have lower 18:0-22:6/N16:0 plasma levels than 64% of the controls. In the light of these results, it is considered that the NAPE quantification may provide, in general, a useful support for PD diagnosis. These tools also allow early diagnosis of PD. The dataset examined included a set of 14 female subjects diagnosed with PD before 50 years of age. Although this group represents a limited sample size, it is important to note that their NAPE plasma levels are much lower than those of 62 age-matched healthy subjects. For this age span, NAPE 18:0-20:4/N18:0 proved to be the best marker for PD, showing an average decrease of about 40% and an AUC for ROC analysis above 0.8 (Figure 3). The corresponding AUC for P16:0-22:6/N18:0 increases from 0.749 for the whole female age range to 0.801 for patients below 50 years of age.

On the basis of these data, the reduced plasma levels of NAPE significantly correlate with the PD diagnosis.

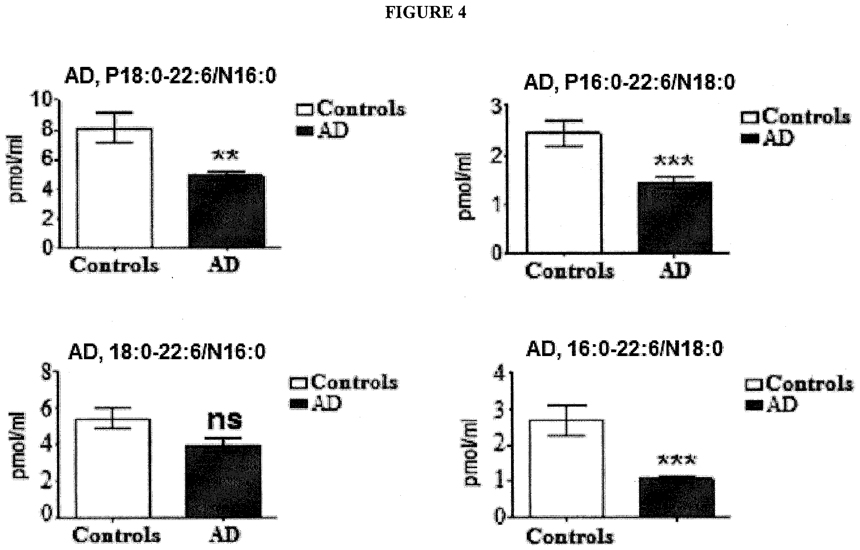

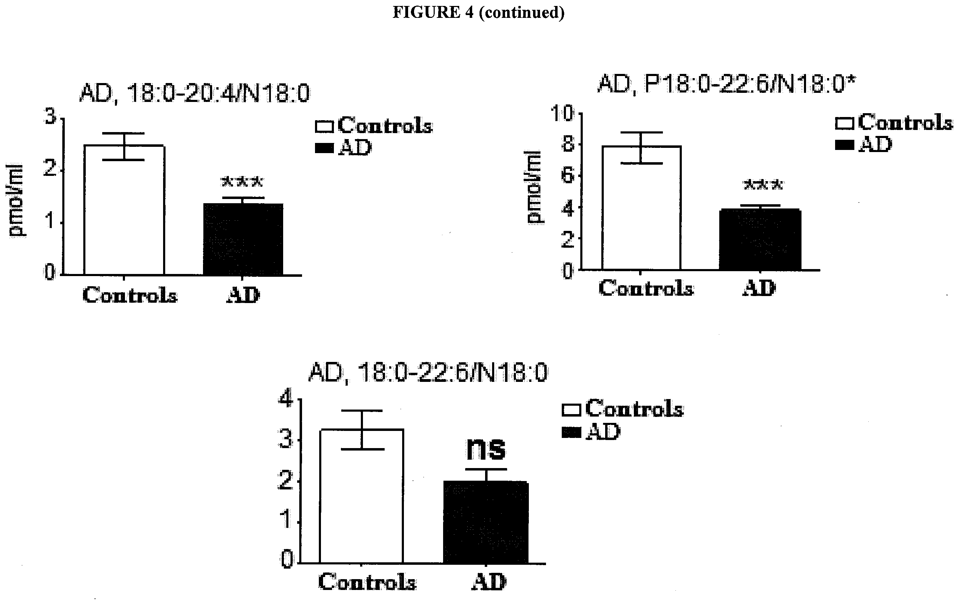

The investigation was then extended to 30 AD patients, and their NAPE plasma levels were measured by comparison with 29 healthy subjects. The results are reported in Figure 4 and Table 3.

**Table 3. Results of comparison of plasma levels of NAPE in healthy subjects and AD patients. The table shows the measured average % change, the P-value calculated with a 2-tailed Mann-Whitney t-test (*) and the AUC deriving from a ROC analysis with 95% confidence (**)**

| | AD SUBJECTS (30) | | | |
|---|---|---|---|---|
| NAPE | Change | P-value* | Significance | AUC** |
| P18:0-22:6-N16:0 | -40% | 0.009 | ** | 0.700 |
| P16:0-22:6-N18:0 | -42% | 0.0001 | *** | 0.776 |
| 16:0-22:6-N18:0 | -61% | 0.001 | *** | 0.751 |
| 18:0-20:4-N18:0 | -45% | 0.0001 | *** | 0.775 |
| P18:0-22:6-N18:0 | -51% | 0.0001 | *** | 0.779 |

As reported in the table, the NAPEs show a significant decrease in the plasma of AD patients. The reduction observed in the AD patients is higher on average than that observed in the PD patients. The NAPEs also proved to be effective markers even when the group of AD patients was clustered by sex, as shown in Table 4 below.

**Table 4. Results of comparison of NAPE plasma levels in healthy subjects and AD patients clustered by sex. The table shows the measured average % change, the P-value calculated with the two-tailed Mann-Whitney t-test (*) and the AUC deriving from a ROC analysis with 95% confidence (**).**

| | FEMALE SUBJECTS (15AD)* | | | | MALE SUBJECTS (15AD)* | | | |
|---|---|---|---|---|---|---|---|---|
| NAPE | Change | P-value* | Significance | AUC** | Change | P-value* | Significance | AUC** |
| P18:0-22:6-N16:0 | -34% | 0.047 | * | 0.719 | -44% | 0.082 | ns | 0.689 |
| P16:0-22:6-N18:0 | -41% | 0.008 | ** | 0.793 | -43% | 0.028 | * | 0.738 |
| 16:0-22:6-N18:0 | -43% | 0.077 | ns | 0.695 | -72% | 0.010 | ** | 0.780 |
| 18:0-20:4-N18:0 | -49% | 0.001 | ** | 0.852 | -40% | 0.075 | ns | 0.693 |
| P18:0-22:6-N18:0 | -42% | 0.004 | ** | 0.814 | -58% | 0.010 | * | 0.778 |

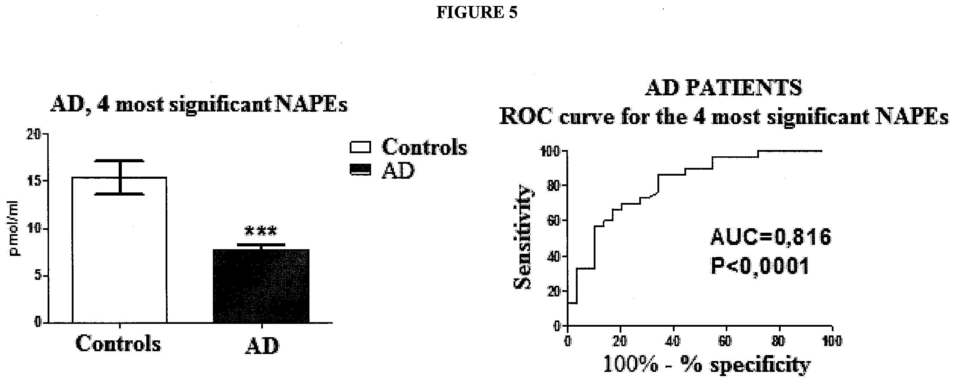

A particularly interesting aspect is that by summing the plasma levels of the NAPEs most significantly decreasing in AD patients, the usefulness of NAPEs as AD markers appears even more evident (Figure 5).

## Claims

1. An *in vitro* method for the diagnosis or prognosis of neurodegenerative disease, or for determining the risk that said disease develops in an individual, said method comprising:
a) determining the concentration of a lipid marker selected from the following N-acylphosphatidylethanolamines (NAPEs): P18:0-22:6/N16:0; P16:0-22:6/N18:0; 18:0-22:6/N16:0; 16:0-22:6/N18:0; 18:0-20:4/N18:0, P18:0-22:6/N18:0, and 18:0-22:6/N18:0, or a combination thereof, in a sample of plasma or serum of said individual, and
b) comparing said concentration to a reference value corresponding to the concentration of lipid marker in the plasma or serum of subjects not suffering from the same neurodegenerative disease,
wherein a decrease in said concentration detected in the plasma or serum sample of the individual under examination with respect to the reference value indicates the presence of neurodegenerative disease, an increased risk of developing it or progression thereof.

2. An *in vitro* method for evaluating the efficacy of a therapeutic treatment of a neurodegenerative disease, said method comprising determining the concentration of a lipid marker selected from the following N-acylphosphatidylethanolamines (NAPEs): P18:0-22:6/N16:0; P16:0-22:6/N18:0; 18:0-22:6/N16:0; 16:0-22:6/N18:0; 18:0-20:4/N18:0, P18:0-22:6/N18:0, and 18:0-22:6/N18:0, or a combination thereof, in a sample of plasma or serum of an individual, wherein a decrease in said concentration of lipid marker during the treatment indicates the progression of the disease and thereby reduced efficacy of the treatment, whereas an increase in said concentration of lipid marker during the treatment indicates a slowing or regression of the disease and thereby increased efficacy of the treatment.

3. Method according to claims 1-2, wherein the neurodegenerative disease is Parkinson's disease.

4. Method according to claims 1-3, wherein the NAPE is selected from P18:0-22:6/N16:0; P16:0-22:6/N18:0; 18:0-22:6/N16:0; 18:0-20:4/N18:0; and 18:0-22:6/N18:0.

5. Method according to claims 1-4, wherein the individual is a female individual and the NAPE is 18:0-20:4/N18:0.

6. Method according to claims 1 and 3-5, wherein the reference value corresponding to the concentration of NAPE in the plasma or serum of subjects not suffering from Parkinson's disease ranges from 1.30 to 3.25 pmol/ml.

7. Method according to claims 1-2, wherein the neurodegenerative disease is Alzheimer's disease.

8. Method according to claims 1-2 and 7, wherein the NAPE is selected from P18:0-22:6/N16:0; P16:0-22:6/N18:0; 16:0-22:6/N18:0; 18:0-20:4/N18:0, and P18:0-22:6/N18:0.

9. Method according to claims 1 and 7-8, wherein the reference value corresponding to the concentration of NAPE in the plasma of subjects not suffering from Alzheimer's disease ranges from 4.0 to 6.0 pmol/ml.

10. Method according to claims 1-9, wherein the amount of lipid marker in the sample under examination is determined with an analytical method based on liquid chromatography and mass spectrometry.

## Patentansprüche

1. *In-vitro*-Verfahren zur Diagnose oder Prognose einer neurodegenerativen Erkrankung oder zur Bestimmung des Risikos, dass sich diese Krankheit bei einem Individuum entwickelt, wobei das Verfahren umfasst:
a) Bestimmung der Konzentration eines Lipidmarkers, ausgewählt aus den folgenden N-Acylphosphatidylethanolaminen (NAPE): P18:0-22:6/N16:0; P16:0-22:6/N18:0; 18:0-22:6/N16:0; 16:0-22:6/N18:0; 18:0-20:4/N18:0, P18:0-22:6/N18:0 und 18:0-22:6/N18:0, oder einer Kombination davon, in einer Plasma- oder Serumprobe der Person, und
b) Vergleich dieser Konzentration mit einem Referenzwert, welcher der Konzentration des Lipidmarkers im Plasma oder Serum von Personen entspricht, die nicht an derselben neurodegenerativen Krankheit leiden,
wobei eine Abnahme der Konzentration, die in der Plasma- oder Serumprobe der untersuchten Person in Bezug auf den Referenzwert erkannt wird, das Vorliegen einer neurodegenerativen Erkrankung, ein erhöhtes Risiko für deren Entwicklung oder deren Fortschreiten anzeigt.

2. *In-vitro*-Verfahren zur Bewertung der Wirksamkeit einer therapeutischen Behandlung einer neurodegenerativen Erkrankung, wobei das Verfahren die Bestimmung der Konzentration eines Lipidmarkers umfasst, ausgewählt aus den folgenden N-Acylphosphatidylethanolaminen (NAPEs): P18:0-22:6/N16:0; P16:0-22:6/N18:0, 18:0-22:6/N16:0; 16:0-22:6/N18.0; 18:0-20:4/N18:0, P18:0-22:6/N18:0 und 18:0-22:6/N18:0, oder einer Kombination davon, in einer Probe von Plasma oder Serum eines Individuums, wobei eine Abnahme der Konzentration des Lipidmarkers während der Behandlung das Fortschreiten der Krankheit und damit eine verringerte Wirksamkeit der Behandlung anzeigt, wohingegen ein Anstieg der Konzentration des Lipidmarkers während der Behandlung eine Verlangsamung oder Rückbildung der Krankheit und damit die Wirksamkeit der Behandlung anzeigt.

3. Verfahren nach Anspruch 1-2, wobei die neurodegenerative Erkrankung Parkinson-Krankheit ist.

4. Verfahren nach den Ansprüchen 1-3, wobei das NAPE ausgewählt ist aus P18:0-22:6/N16:0; P16:0-22:6/N18:0; 18:0-22:6/N16:0; 18:0-20:4/N18:0 und 18:0-22:6/N18:0.

5. Verfahren nach den Ansprüchen 1-4, wobei das Individuum ein weibliches Individuum ist und das NAPE 18:0-20:4/N18:0 ist.

6. Verfahren nach den Ansprüchen 1 und 3-5, wobei der Referenzwert, welcher der Konzentration von NAPE im Plasma oder Serum von Personen entspricht, die nicht an der Parkinson-Krankheit leiden, im Bereich von 1,30 bis 3,25 pmol/ml liegt.

7. Verfahren nach Anspruch 1-2, wobei die neurodegenerative Krankheit Alzheimer-Krankheit ist.

8. Verfahren nach den Ansprüchen 1-2 und 7, wobei das NAPE ausgewählt ist aus P18:0-22:6/N16:0; P16:0-22:6/N18:0; 16:0-22:6/N18:0; 18:0-20:4/N18:0 und P18:0-22:6/N18:0.

9. Verfahren nach den Ansprüchen 1 und 7-8, wobei der Referenzwert, welcher der Konzentration von NAPE im Plasma von Personen entspricht, die nicht an der Alzheimer-Krankheit leiden, im Bereich von 4,0 bis 6,0 pmol/ml liegt.

10. Verfahren nach einem der Ansprüche 1-9, wobei die Menge des Lipidmarkers in der untersuchten Probe mit einem analytischen Verfahren auf der Basis von Flüssigchromatographie und Massenspektrometrie bestimmt wird.

## Revendications

1. Procédé *in vitro* pour le diagnostic ou le pronostic d'une maladie neurodégénérative ou pour la détermination du risque que développe ladite maladie chez un individu, ledit procédé comprenant :
a) la détermination de la concentration d'un marqueur lipidique choisi parmi les N-acylphosphatidyléthanolamines (NAPEs) suivantes : P18:0-22:6/N16:0; P16:0-22:6/N18:0; 18:0-22:6/N16:0; 16:0-22:6/N18:0; 18:0-20:4/N18:0 ; P18:0-22:6/N18:0 ; et 18:0-22:6/N18:0, ou une combinaison de ces dernières, dans un échantillon de plasma ou de sérum dudit individu, et
b) la comparaison de ladite concentration à une valeur de référence correspondant à la concentration du marqueur lipidique dans le plasma ou le sérum de sujets qui ne souffrent pas de la même maladie neurodégénérative,
dans lequel une diminution dans ladite concentration détectée dans l'échantillon de plasma ou de sérum de l'individu soumis à l'examen par rapport à la valeur de référence indique la présence d'une maladie neurodégénérative, un risque accru de la développer ou sa progression.

2. Procédé *in vitro* destiné pour l'évaluation de l'efficacité d'un traitement thérapeutique d'une maladie neurodégénérative, ledit procédé comprenant la détermination de la concentration d'un marqueur lipidique choisi parmi les N-acylphosphatidyléthanolamines (NAPEs) suivantes : P18:0-22:6/N16:0 ; P16:0-22:6/N18:0; 18:0-22:6/N16:0; 16:0-22:6/N18:0; 18:0-20:4/N18:0 ; P18:0-22:6/N18:0 ; et 18:0-22:6/N18:0, ou une combinaison de ces dernières, dans un échantillon de plasma ou de sérum d'un individu, dans lequel une diminution dans ladite concentration du marqueur lipidique au cours du traitement indique la progression de la maladie, et ainsi une réduction d'efficacité du traitement, tandis qu'une augmentation dans ladite concentration du marqueur lipidique au cours du traitement indique un ralentissement ou une régression de la maladie, et ainsi une augmentation de l'efficacité du traitement.

3. Procédé selon les revendications 1 à 2, dans lequel la maladie neurodégénérative est la maladie de Parkinson.

4. Procédé selon les revendications 1 à 3, dans lequel la NAPE est choisie parmi P18:0-22:6/N16:0; P16:0-22:6/N18:0; 18:0-22:6/N16:0; 18:0-20:4/N18:0 et 18:0-22:6/N18:0.

5. Procédé selon les revendications 1 à 4, dans lequel l'individu est un individu de sexe féminin et la NAPE est 18:0-20:4/N18:0.

6. Procédé selon les revendications 1 et 3 à 5, dans lequel la valeur de référence correspondant à la concentration de la NAPE dans le plasma ou le sérum de sujets qui ne souffrent pas de la maladie de Parkinson se situe dans la plage de 1,30 à 3,25 pmol/ml.

7. Procédé selon les revendications 1 à 2, dans lequel la maladie neurodégénérative est la maladie d'Alzheimer.

8. Procédé selon les revendications 1 à 2 et 7, dans lequel la NAPE est choisie parmi P18:0-22:6/N16:0 ; P16:0-22:6/N18:0 ; 16:0-22:6/N18:0 ; 18:0-20:4/N18:0 et P18:0-22:6/N18:0.

9. Procédé selon les revendications 1 et 7 à 8, dans lequel la valeur de référence correspondant à la concentration de la NAPE dans le plasma de sujets qui ne souffrent pas de la maladie d'Alzheimer se situe dans la plage de 4,0 à 6,0 pmol/ml.

10. Procédé selon les revendications 1 à 9, dans lequel la quantité du marqueur lipidique dans l'échantillon soumis à l'examen est déterminée avec un procédé analytique qui se base sur une chromatographie en phase liquide et une spectrométrie de masse.
